(19) Europäisches Patentamt
European Patent Office
Office européen des brevets

(11) Veröffentlichungsnummer: **0 126 483 B1**

(12) # EUROPÄISCHE PATENTSCHRIFT

(45) Veröffentlichungstag der Patentschrift: **22.05.91**

(51) Int. Cl.5: **A61K 7/32**

(21) Anmeldenummer: **84105789.6**

(22) Anmeldetag: **21.05.84**

Die Akte enthält technische Angaben, die nach dem Eingang der Anmeldung eingereicht wurden und die nicht in dieser Patentschrift enthalten sind.

(54) **Deodorant.**

(30) Priorität: **24.05.83 DE 3318789**

(43) Veröffentlichungstag der Anmeldung:
**28.11.84 Patentblatt 84/48**

(45) Bekanntmachung des Hinweises auf die Patenterteilung:
**22.05.91 Patentblatt 91/21**

(84) Benannte Vertragsstaaten:
**AT BE CH DE FR GB IT LI LU NL SE**

(56) Entgegenhaltungen:

| | |
|---|---|
| **CH-B- 429 624** | **DE-A- 2 531 260** |
| **DE-A- 2 604 554** | **DE-A- 3 009 543** |
| **DE-B- 1 238 619** | **FR-A- 2 224 127** |
| **FR-A- 2 251 310** | **FR-A- 2 353 284** |

(73) Patentinhaber: **Eduard Gerlach GmbH Chemische Fabrik**
**Bäckerstrasse 6-8**
**W-4990 Lübbecke(DE)**

(72) Erfinder: **Detert, Ernst-Rolf, Dr. Dipl.-Chem.**
**Schützenstrasse 2**
**W-4990 Lübbecke(DE)**

(74) Vertreter: **Rücker, Wolfgang, Dipl.-Chem.**
**Alte Dorfstrasse 16**
**W-3160 Lehrte OT Arpke(DE)**

EP 0 126 483 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Deodorant.

Präparate zur Verhütung von Körpergeruch sind aus der Reihe der Körperpflegemittel nicht mehr wegzudenken. Sie werden in Form von Sprays, Lotions, Cremes, Roll-on-Produkten, Stiften und Pudern verwendet. Man unterscheidet zwei Möglichkeiten, die zum Ziel führen:

Die Antiperspirantien unterbinden ganz oder teilweise die Schweißbildung durch Applikation adstringierender Stoffe. Hauptsächlich sind es Aluminiumsalze organischer oder anorganischer Säuren, die in dieser Präparate-Gruppe als Wirkstoffe eingesetzt werden.

Die Deodorantien enthalten häufig als Wirkstoffe bakterzizide Substanzen phenolischer Art. Sie vernichten die bakterielle Hautflora, welche den an sich geruchlosen Schweiß zu unangenehm riechenden Produkten zersetzt.

Antiperspirantien und Deodorantien werden auch kombiniert.

Die ohne Zweifel vorhandene Wirksamkeit beider Produktgruppen zur Verhütung von Körpergeruch wird benachteiligt durch immer wieder auftretende Hautreizungen, die sich von Juckreiz bis zu schmerzhaften Hautrötungen erstrecken, besonders dann, wenn das betreffende Präparat über einen langen Zeitraum täglich angewendet wird. Außerdem wird der Geruch nach "Desinfektion", der unterschwellig vielen der verwendeten phenolischen Substanzen eigen ist, als störend empfunden. Da es aber Sinn derartiger Präparate ist, sie täglich anzuwenden, wurde nach anderen Wegen der Desodorierung gesucht, die hautfreundlicher und verträglicher sind.

Man fand sie u. a. im Einsatz von Enzymblockern, die nicht die Mikroflora der Haut vernichten, sondern die Wirkung der von den Bakterien ausgeschiedenen Enzyme aufheben. In Anwesenheit derartiger Substanzen verlieren die Enzyme die Fähigkeit, beispielsweise Ester zu spalten oder dehydrierende Aktivitäten zu entfalten. Salze der Malonsäuren und Metallchelate der ß-Dikarbonylverbindungen sollen der Zersetzung der im Schweiß vorhandenen Eiweißkörper entgegenwirken. (Jellinek, Kosmetologie, Hüthig Verlag 1967).

In der deutschen Auslegeschrift 24 18 338 werden Ester der Zitronensäure oder der Azetylzitronensäure beschrieben, die sich durch "außerordentliche desodorierende Wirksamkeit" auszeichnen. Genannt werden insbesondere deren Triester mit aliphatischen oder alicyclischen Alkoholen mit 1 - 6 Kohlenstoffatomen.

Aufgabe der vorliegenden Erfindung ist es, eine weitere, umfangreiche Wirkstoffgruppe für Deodorantien vorzuschlagen, deren Substanzen nicht nur eine ausgezeichnete Wirkung besitzen, sondern gut hautverträglich sind, nicht zu Hautreizungen, Rötungen oder gar Entzündungen führen, selbst dann nicht, wenn die Anwendung täglich oder mehrmals täglich erfolgt und die geschmacklich nicht irritieren. Außerdem enthält diese Wirkstoffgruppe viele Verbindungen, die in der Natur vorkommen.

Gelöst wird diese Aufgabe durch die Verwendung der in den Patentansprüchen 1 bis 8 beschriebenen Verbindungen und Zusammensetzungen.

Desodorierende kosmetische Zusammensetzungen ähnlicher Art sind aus der DE-OS 30 09 543 bekannt, die α-verzweigte Alkansäuren der allgemeinen Formel

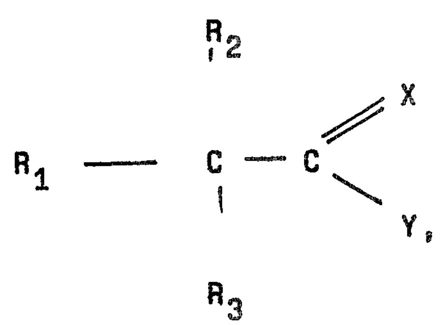

enthalten in der $R_1$ für einen geradkettigen Alkrylrest mit 1 - 12 Kohlenstoffatomen oder den Rest

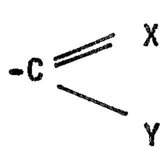

$R_2$ für einen geradkettigen Alkylrest mit 1 - 6 Kohlenstoffatomen, $R_3$ für ein Wasserstoffatom oder einen garadkettigen Alkylrest mit 1 - 6 Kohlenstoffatomen, X für Sauerstoff, Y für -OH, $-OR_4$, $-NR_5R_6$, wobei $R_4$ einen Alkylrest mit 1 - 10 Kohlenstoffatomen oder einen Aralkylrest und $R_5$ und $R_6$ unabhängig voneinanderWasserstoff, einen Alkylrest mit 1 - 12 Kohlenstoffatomen, einen Hydroxyalkylrest mit 2 - 4 Kohlenstoffatomen, einen Alkoxyalkylrest, einen Aryl- oder Aralkylrest bedeuten oder zussmmen mit dem Stickstoffatom einen heterocyclischen Rind bilden, beziehungsweise

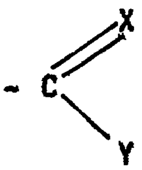

auch für eine Nitrilgruppe stehen.
Diese Derivate der α verzweigten Alkansnsäure sind zwar ähnlich strukturiert, aber die Verbindungen der Erfindung, bei den es sich um unverzweigte Amide oder N-substituierte Amide ein- oder mehrbasiger Karbonsäure handelt zeigen gleichgute, zum Teil sogar bessere desodorierende Wirkung und haben den Vorteil einfacher in der Herstellung zu sein.

In Verfolg des Erfindungsgedankens hat sich als besonders kräftiger Wirkstoff Essigsäuremonoethanolamid erwiesen, der in Anteilen von 3 bis 5 % in einem desodorierenden Präpärat vor der Bildung unangenehmer Zersetzungsprodukte sowohl des Achsel- als auch des Fußschweißes bewahrt.

Die gleiche Eigenschaft haben die Ethanolamide der Propionsäure und der Milchsäure. Erfolgreich getestet wurden weiter die Ethanolamide der Malonsäure, Bernsteinsäure, Glutarsäure, Adipinsäure, Zitronensäure und Weinsäure.

Eine weitere überraschend wirksame desodorierende Substanz mit hervorragender Hautfreundlichkeit ist das zu den B-Vitaminen gehörende Panthenol . Als n-Propanolamid der 1,3 Dihydroxi 2,2 Dimethylbuttersäure wird Panthenol von der erfindungsgemäßen Gruppe der Amide organischer Säuren, mit erfaßt. Schon in einer 3 %igen Lösung in einem Alkohol/Wassergemisch bewirkt Panthenol eine befriedigende Desodorierung des Achsel- und Fußschweißes. Ein Anteil von 5 % in einem Alkohol/Wassergemisch wirkt optimal auch bei stark transpirierenden Personen. Offenbar wird der an sich wasserlösliche Wirkstoff auf der Haut fixiert, so daß die langanhaltende Wirkung auch bei erheblicher Flüssigkeitsausscheidung erhalten bleibt. Die spezielle Wirksamkeit des Panthenols als Deodorant ist bisher nicht beschrieben worden.

Auch das Diamid der Kohlensäure, der Harnstoff kann mit bestem Erfolg für sich allein oder in Kombination mit den vorstehend beschriebenen Wirkstoffen als desodorierender Stoff verwendet werden. In der Arbeit E. Redenz "Der Harnstoff als keimtötendes Desodorans und seine Bedeutung für die Wundheilung" wird auf die gute Eignung dieses Naturstoffes als Desodorierungsmittel von übelriechenden Wunden hingewiesen. Die Eignung des Harnstoffes zur Verhütung der Zersetzung des Schweißes ist bisher nicht bekannt.

Sehr wirksam ist ß-Hydroxiethylharnstoff. Als "Hautfeuchthalter" ist hydroxyalkylierter Harnstoff (DOS 2703185) bekannnt. Wirksam im erfindungsg. Sinne sind auch die Harnstoffabkömmlinge Biuret, Urethan, Hippursäure, Malonylharnstoff(Barbitursäure) und deren Ester und deren Salze, insbesondere die Monoethanolamin-, Diethanolamin- und Triethanolaminsalze.

Zur Herstellung der Säureamide können mit Erfolg nur solche Amine und Säuren eingesetzt werden, die keinen unangenehmen Geruch aufweisen. Bei eventuell erfolgender Spaltung der Substanz, würden sie sich sonst durch starken und auch haftenden Eigengeruch störend bemerkbar machen.

Im Sinne der Erfindung gut brauchbar haben sich folgende Aminkomponenten erwiesen: Ammoniak, Monoethanolamin, Diethanolamin und Propanolamin. Mit dieser Aufzählung sind keinesfalls alle brauchbaren Amine erfaßt.

So kommen z. B. auch die natürlichen Aminosäuren als Amidbildner in Frage. Deren Kondensationsprodukte mit geeigneten Säuren z. B. N-Acetylglycin (Acetursäure), dessen Ester mit niederen Alkoholen und dessen Salze, insbesondere die Alkylolaminsalze, sind gute Wirkstoffe für Desodorentien.

Auffällig und für kosmetische Zwecke beachtenswert ist eine zusätzlich von vielen Probanden festgestellte langanhaltend kühlende Wirkung, insbesondere der Ethanolamide niederer mono-, di- und mehrbasischer Carbonsäuren. Die überraschende Eigenschaft macht die beschriebene Wirkstoffgruppe besonders für Fußpflegepräparate bedeutungsvoll, zumal der Kühleffekt praktisch 12 Stunden und länger als milde Frische empfunden wird.

Die erfindungsgemäßen Amide geeigneter Carbonsäuren werden folgendermaßen in kosmetischen

Produkten verwendet (Rahmenrezepturen):

I

Desodorierende und kühlende Lotion für Füße

| | |
|---|---|
| Isopropanol | 40,0 Gewichtsteile |
| Essigsäuremonoethanolamid | 5,0 Gewichtsteile |
| Fungizide Wirkstoffe | 0,5 Gewichtsteile |
| 1,2 Propylenglykol | 2,0 Gewichtsteile |
| Duftstoffe | 0,5 Gewichtsteile |
| Wasser, enthärtet | 52,0 Gewichtsteile |
| | 100,0 Gewichtsteile |

II

Desodorierender Spray für Pumpzerstäuber

| | |
|---|---|
| Ethanol | 40,0 Gewichtsteile |
| Harnstoff | 2,0 Gewichtsteile |
| Essigsäuremonoethanolamid | 2,0 Gewichtsteile |
| Panthenol | 2,0 Gewichtsteile |
| Duftstoff | 0,2 Gewichtsteile |
| Wasser, enthärtet | 53,8 Gewichtsteile |
| | 100,0 Gewichtsteile |

III

Desodorierender Spray für Pumpzerstäuber

| | |
|---|---|
| Ethanol | 40,0 Gewichtsteile |
| Milchsäuremonoethanolamid | 2,5 Gewichtsteile |
| Panthenol | 2,5 Gewichtsteile |
| Duftstoff | 0,2 Gewichtsteile |
| Wasser, enthärtet | 54,8 Gewichtsteile |
| | 100,0 Gewichtsteile |

IV

Desodorierender Spray

| | |
|---|---|
| Propionsäuremonoethanolamid | 10,0 Gewichtsteile |
| Isopropylpalmitat | 1,0 Gewichtsteile |
| Propylenglykol | 2,0 Gewichtsteile |
| Duftstoffe | 0,5 Gewichtsteile |
| Ethanol | 26,5 Gewichtsteile |
| Treibmittel | 60,0 Gewichtsteile |
| | 100,0 Gewichtsteile |

V

Desodorierender Fußspray (stark kühlend)

| | |
|---|---|
| Malonsäurediethanolamid | 5,0 Gewichtsteile |
| Fungizide Wirkstoffe | 0,5 Gewichtsteile |
| Duftstoffe | 0,5 Gewichtsteile |
| Propylenglykol | 5,0 Gewichtsteile |
| Isopropylalkohol | 29,0 Gewichtsteile |
| Treibmittel | 60,0 Gewichtsteile |
| | 100,0 Gewichtsteile |

VI

Desodorierende und kühlende Fußkrem

| | |
|---|---|
| Cremebasis Typ O/W | 93,5 Gewichtsteile |
| Milchsäuremonoethanolamid | 2,5 Gewichtsteile |
| Essigsäuremonoetnanolamid | 2,5 Gewichtsteile |
| Duftstoffe | 1,0 Gewichtsteile |
| Fungizide Wirkstoffe | 0,5 Gewichtsteile |
| | 100,0 Gewichtsteile |

VII

Desodorierender Stift

| | |
|---|---|
| Stiftmasser bekannter Art | 94,5 Gewichtsteile |
| Essigsäuremonoethanolamid | 2,5 Gewichtsteile |
| Panthenol | 2,5 Gewichtsteile |
| Duftstoffe | 0,5 Gewichtsteile |
| | 100,0 Gewichtsteile |

VIII

Desodorierender, kühlender Puder

| | |
|---|---|
| Malonsäurebisethanolamid | 2,5 Gewichtsteile |
| Panthenol | 2,5 Gewichtsteile |
| Duftstoffe | 0,5 Gewichtsteile |
| Pudergrundlage | 94,5 Gewichtsteile |
| | ------ |
| | 100,0 Gewichtsteile |
| | ====== |

IX

Badesalz mit stark desodorierenden und Hornhaut erweichenden Eigenschaften

| | |
|---|---|
| Harnstoff | 85,0 Gewichtsteile |
| Soda kalciniert | 10,0 Gewichtsteile |
| Comperlan LS | 1,0 Gewichtsteile |
| Texapon WW 99 | 1,0 Gewichtsteile |
| ätherische Öle | 3,0 Gewichtsteile |
| | ------ |
| | 100,0 Gewichtsteile |
| | ====== |

Die Wirksamkeit der in der Beschreibung genannte Verbindungen wurde folgendermaßen ermittelt:

1) Die Versuchspersonen, 10 männliche und 10 weibliche Probanden, benutzten zur Körperreinigung ein von bakteriziden und fungiziden Stoffen freies Duschbad. Die linke Achselhöhle und der linke Fuß, insbesondere die Partien zwischen den Zehen, wurden mit einem Präparat gemäß den Beispielen behandelt. Die rechte Achselhöhle und der rechte Fuß wurden nicht behandelt. Die Prüfung erfolgte nach 12 und 24 Stunden durch die Versuchsansteller, indem jeweils ein geruchsfreies Mullkissen aus 4 Lagen Verbandmull mit den behandelten und unbehandelten Stellen in intensive Berührung durch Reiben gebracht wurde. Anschließend prüften die Tester den Geruch der Kissen selbst. Dabei wurde festgestellt, daß an den mit den oben beschriebenen Präparaten imprägnierten Stellen sich keine Schweißzersetzung bemerkbar machte, wogegen die unbehandelten Stellen deutlich Schweißzersetzung aufwiesen.

2) 7 weibliche und 7 männliche Versuchsteilnehmer bekamen je ein Präparat gegen Achselschweiß und ein Präparat gegen Fußschweiß, hergestellt gem. der Beschreibung, zur Anwendung. Sie wurden verpflichtet, kein anderes desodorierendes Präparat, sei es ein kosmetisches Spezialpräparat oder eine sogenannte "Deo-Seife" zu benutzen. Alle 14 Tester betonten die sehr gute desodorierende Wirkung der von ihnen erprobten Präparate und lobten die gute Verträglichkeit. Sie stellten alle eine wirkungsdauer bis zu 24 Stunden fest.

Als weitere Verbindung, die im erfindungsgemäßen Sinn eingesetzt werden können, seien genannt: Harnstoff, Diethanolamid der Propionsäure und der Milchsäure, der Maleinsäure, der Bernsteinsäure, der Glutarsäure, der Adipinsäure, der Zitronensäure und der Weinsäure, das N-Propanolamid der 1,3-Dihydroxy-2,2-Dimethylbuttersäure (Panthenol), β-Hydroxyethylharnstoff, Biuret, Urethan, Hippursäure, Malonylharnstoff (Barbitursäure) und Acetursäure, deren Ester und Salze.

**Ansprüche**

1. Verwendung von Essigsäuremonoethanolamid, Diethanolamid der Propionsäure und der Milchsäure, der Maleinsäure, der Bernsteinsäure, der Glutarsäure, der Adipinsäure, der Zitronensäure und der Weinsäure, das n-Propanolamid der 2,4-Dihydroxy-3,3-dimethylbuttersäure (Panthenol), Harnstoff, β-Hydroxyethylharnstoff, Biuret, Urethan, Hippursäure, Malonyharnstoff (Barbitursäure) und Acetursäure, deren Ester und Salze einzeln oder im Gemisch als desodorierend wirkender Stoff in Deodorantien.

2. Verwendung von desodorierend wirkenden Stoffen nach Anspruch 1, in einem als Lotion bereiteten Deodorant folgender Zusammensetzung:

| | |
|---|---|
| Isopropanol | 40,0 Gewichtsteile |
| Essigsäuremonoethanolamid | 5,0 Gewichtsteile |
| Fungizide Wirkstoffe | 0,5 Gewichtsteile |
| 1,2 Propylenglykol | 2,0 Gewichtsteile |
| Duftstoffe | 0,5 Gewichtsteile |
| Wasser, enthärtet | 52,0 Gewichtsteile |

3. Verwendung von desodorierend wirkenden Stoffen nach Anspruch 1, in einem als Spray bereiteten Deodorant folgender Zusammensetzung:

| | |
|---|---|
| Ethanol | 40,0 Gewichtsteile |
| Harnstoff | 2,0 Gewichtsteile |
| Essigsäuremonoethanolamid | 2,0 Gewichtsteile |
| Panthenol | 2,0 Gewichtsteile |
| Duftstoff | 0,2 Gewichtsteile |
| Wasser, enthärtet | 53,8 Gewichtsteile |

4. Verwendung von desodorierend wirkenden Stoffen nach Anspruch 1, in einem als Spray bereiteten Deodorant folgender Zusammensetzung:

| | |
|---|---|
| Propionsäuremonoethanolamid | 10,0 Gewichtsteile |
| Isopropylpalmitat | 1,0 Gewichtsteile |
| Propylenglykol | 2,0 Gewichtsteile |
| Duftstoffe | 0,5 Gewichtsteile |
| Ethanol | 26,5 Gewichtsteile |
| Treibmittel | 60,0 Gewichtsteile |

5. Verwendung von desodorierend wirkenden Stoffen nach Anspruch 1, in einem als Fußcreme bereiteten Deodorant folgender Zusammensetzung:

| | |
|---|---|
| Cremebasis Typ O/W | 93,5 Gewichtsteile |
| Milchsäuremonoethanolamid | 2,5 Gewichtsteile |
| Essigsäuremonoethanolamid | 2,5 Gewichtsteile |
| Duftstoffe | 1,0 Gewichtsteile |
| Fungizide Wirkstoffe | 0,5 Gewichtsteile |

6. Verwendung von desodorierend wirkenden Stoffen nach Anspruch 1, in einem als Stift bereiteten Deodorant folgender Zusammensetzung:

| | |
|---|---|
| Stiftmasser bekannter Art | 94,5 Gewichtsteile |
| Essigsäuremonoethanolamid | 2,5 Gewichtsteile |
| Panthenol | 2,5 Gewichtsteile |
| Duftstoffe | 0,5 Gewichtsteile |

7. Verwendung von desodorierend wirkenden Stoffen nach Anspruch 1, in einem als Puder bereiteten Deodorant folgender Zusammensetzung:

| | |
|---|---|
| Malonsäurebisethanolamid | 2,5 Gewichtsteile |
| Panthenol | 2,5 Gewichtsteile |
| Duftstoffe | 0,5 Gewichtsteile |
| Pudergrundlage | 94,5 Gewichtsteile |

8. Verwendung von desodorierend wirkenden Stoffen nach Anspruch 1, in einem als Badesalz bereiteten Deodorant folgender Zusammensetzung:

| | |
|---|---|
| Harnstoff | 85,0 Gewichtsteile |
| Soda kalciniert | 10,0 Gewichtsteile |
| Comperlan LS | 1,0 Gewichtsteile |
| Texapon WW 99 | 1,0 Gewichtsteile |
| ätherische Öle | 3,0 Gewichtsteile |

## Claims

1. Use of acetic acid monoethanolamide, diethanolamide of propionic acid of lactic acid, of maleic acid, of succinic acid, of glutaric acid, of adipic acid, of citric acid and of tartaric acid, the n-propanolamide of 2,4-dihydroxy-3,3-dimethyl butyric acid (panthenol), urea, $\beta$-hydroxy-ethyl urea, biuret, urethane, hippuric acid, malonylurea (barbituric acid) and aceturic acid, the esters and salts thereof singly or in

mixture, as substance having a deodorising effect, in deodorants.

2. Use of substances having a deodorising effect according to Claim 1, in a deodorant prepared as a lotion, of the following composition:

| | |
|---|---|
| isopropanol | 40.0 parts by weight |
| acetic acid monethanolamide | 5.0 parts by weight |
| fungicidally active substances | 0.5 parts by weight |
| 1,2 propylene glycol | 2.0 parts by weight |
| perfumes | 0.5 parts by weight |
| water, softened | 52.0 parts by weight. |

3. Use of substances having a deodorising effect according to Claim 1, in a deodorant prepared as a spray, of the following composition:

| | |
|---|---|
| ethanol | 40.0 parts by weight |
| urea | 2.0 parts by weight |
| acetic acid monoethanolamide | 2.0 parts by weight |
| panthenol | 2.0 parts by weight |
| perfume | 0.2 parts by weight |
| water, softened | 53.8 parts by weight. |

4. Use of substances having a deodorising effect according to Claim 1, in a deodorant prepared as a spray, of the following composition :

| | |
|---|---|
| propionic acid monethanolamide | 10.0 parts by weight |
| isopropyl palmitate | 1.0 part by weight |
| propylene glycol | 2.0 parts by weight |
| perfumes | 0.5 parts by weight |
| ethanol | 26.5 parts by weight |
| propellant | 60.0 parts by weight. |

5. Use of substances having a deodorising effect according to Claim 1, in a deodorant prepared as a foot cream, of the following composition :

| | |
|---|---|
| cream base type O/W | 93.5 parts by weight |
| lactic acid monoethanolamide | 2.5 parts by weight |
| acetic acid monoethanolamide | 2.5 parts by weight |
| perfumes | 1.0 part by weight |
| fungicidally active substances | 0.5 parts by weight |

6. Use of substances having a deodorising effect according to Claim 1, in a deodorant prepared as a stick, of the following composition :

| | |
|---|---|
| stick composition of known kind | 94.5 parts by weight |
| acetic acid monoethanolamide | 2.5 parts by weight |
| panthenol | 2.5 parts by weight |
| perfumes | 0.5 parts by weight. |

7. Use of substances having a deodorising effect according to Claim 1, in a deodorant prepared as a powder, of the following composition :

| | |
|---|---|
| malonic acid bisethanol-amide | 2.5 parts by weight |
| panthenol | 2.5 parts by weight |
| perfumes | 0.5 parts by weight |
| powder base | 94.5 parts by weight. |

8. Use of substances having a deodorising effect according to Claim 1, in a deodorant prepared as a bath salt, of the following composition :

| | |
|---|---|
| urea | 85.0 parts by weight |
| soda, calcined | 10.0 parts by weight |
| "Comperlan LS" | 1.0 part by weight |
| "Texapon WW 99" | 1.0 part by weight |
| ethereal oils | 3.0 parts by weight. |

**Revendications**

1. Utilisation du monoéthanolamide de l'acide acétique, du diéthanolamide de l'acide propionique et de l'acide lactique, de l'acide maléique, de l'acide succinique, de l'acide glutarique, de l'acide adipique, de l'acide citrique et de l'acide tartrique, du n-propanolamide de l'acide 2,4-dihydroxy-3,3-diméthylbutyrique (panthénol), de l'urée, de la $\beta$-hydroxyéthylurée, du biuret, de l'uréthane, de l'acide hippurique, de la malonylurée (acide barbiturique) et de l'acide acéturique, de leurs esters et de leurs sels, séparément ou en mélange comme substances à effet désodorisant dans les déodorants.

2. Utilisation de substances à effet désodorisant selon la revendication 1 dans lesquelles le déodorant préparé comme lotion présente la composition suivante:

| | |
|---|---|
| Isopropanol | 40,0 parties en poids |
| Monoéthanolamide de l'acide acétique | 5,0 parties en poids |
| Substance active fongicide | 0,5 partie en poids |
| 1,2-propylèneglycol | 2,0 parties en poids |
| Aromates | 0,5 partie en poids |
| Eau adoucie | 52,0 parties en poids |

3. Utilisation de substances à action désodorisante selon la revendication 1 dans un déodorant préparé sous forme de spray et ayant la composition suivante :

| | |
|---|---|
| Ethanol | 40,0 parties en poids |
| Urée | 2,0 parties en poids |
| Monoéthanolamide de l'acide acétique | 2,0 parties en poids |
| Panthénol | 2,0 parties en poids |
| Aromates | 0,2 partie en poids |
| Eau adoucie | 53,8 parties en poids |

4. Utilisation de substances à action désodorisante selon la revendication 1 dans un déodorant préparé sous forme de spray ayant la composition suivante:

| | |
|---|---|
| Monoéthanolamide de l'acide propionique | 10,0 parties en poids |
| Palmitate d'isopropyle | 1,0 partie en poids |
| Propylèneglycol | 2,0 parties en poids |
| Aromates | 0,5 partie en poids |
| Ethanol | 26,5 parties en poids |
| Propulseur | 60,0 parties en poids |

5. Utilisation de substances à action désodorisante selon la revendication 1 dans un déodorant préparé sous forme de crème pour les pieds ayant la composition suivante :

| | |
|---|---|
| Crème de base type O/W | 93,5 parties en poids |
| Monoéthanolamide de l'acide lactique | 2,5 parties en poids |
| Monoéthanolamide de l'acide acétique | 2,5 parties en poids |
| Aromates | 1,0 partie en poids |
| Substance active fongicide | 0,5 partie en poids |

6. Utilisation de substances à action désodorisante selon la revendication 1 dans un déodorant préparé sous forme de stick ayant la composition suivante:

| | |
|---|---|
| Masse de stick de type connu | 94,5 parties en poids |
| Monoéthanolamide de l'acide acétique | 2,5 parties en poids |
| Panthénol | 2,5 parties en poids |
| Aromates | 0,5 partie en poids |

7. Utilisation de substances à action désodorisante selon la revendication 1 dans un déodorant préparé sous forme de poudre ayant la composition suivante:

| | |
|---|---|
| Bis-éthanolamide de l'acide malonique | 2,5 parties en poids |
| Panthénol | 2,5 parties en poids |
| Aromates | 0,5 partie en poids |
| Poudre de base | 94,5 parties en poids |

8. Utilisation de substances à action désodorisante selon la revendication 1 dans un déodorant préparé sous forme de sels de bains ayant la composition suivante :

| | |
|---|---|
| Urée | 85,0 parties en poids |
| Soude calcinée | 10,0 parties en poids |
| Comperlan LS | 1,0 partie en poids |
| Texapon WW 99 | 1,0 partie en poids |
| Huile éthérée | 3,0 parties en poids |